(19)

Europäisches
Patentamt
European
Patent Office
Office européen
des brevets

(11)  **EP 1 927 851 A2**

(12)  **EUROPEAN PATENT APPLICATION**

(43) Date of publication:
   **04.06.2008  Bulletin 2008/23**

(51) Int Cl.:
   **G01N 27/327** *(2006.01)*   **C12Q 1/00** *(2006.01)*

(21) Application number: **07003914.4**

(22) Date of filing: **26.02.2007**

(84) Designated Contracting States:
   **AT BE BG CH CY CZ DE DK EE ES FI FR GB GR HU IE IS IT LI LT LU LV MC NL PL PT RO SE SI SK TR**
   Designated Extension States:
   **AL BA HR MK RS**

(30) Priority: **30.11.2006  KR 20060120043**

(71) Applicant: **Infopia Co., Ltd.**
   **Dongan-gu**
   **Anyang-si, Gyeonggi-do (KR)**

(72) Inventors:
   • **Bae, Byeong-woo**
    **Anyang-si**
    **Gyeonggi-do (KR)**
   • **Lee, Sung-dong**
    **Anyang-si**
    **Gyeonggi-do (KR)**

   • **Suk, Hong-seong**
    **Anyang-si**
    **Gyeonggi-do (KR)**
   • **Yoo, Jin-a**
    **Gyeonggi-do (KR)**
   • **Kim, Ji-su**
    **Anyang-si**
    **Gyeonggi-do (KR)**
   • **Yang, Jeong-yun**
    **Haman-gun**
    **Gyeongsangnam-do (KR)**

(74) Representative: **Grünecker, Kinkeldey,**
   **Stockmair & Schwanhäusser**
   **Anwaltssozietät**
   **Leopoldstrasse 4**
   **80802 München (DE)**

(54)  **Biosensor**

(57)   A biosensor measuring an analyte contained in a sample is disclosed, including: a lower insulating substrate having formed thereon a working electrode and a reference electrode connected to lead terminals through leads, and an enzyme reactant layer formed on the electrodes to react with the analyte contained in the sample; a spacer which is interposed between the lower substrate and an upper substrate, is attached to the lower and upper substrates, and has a sample guide area to guide the sample to reach to the electrodes through the enzyme reactant layer; and an upper insulating substrate which faces the lower substrate through the spacer, where a dummy electrode is formed on the lower substrate, the dummy electrode being separated from the working electrode and the reference electrode, fixing the enzyme reactant layer, and being not connected to the leads.

FIG.1

**Description**

CROSS REFERENCE TO RELATED APPLICATION

This application claims the benefit of and priority to Korean Patent Application No. 2006-120043, filed on November 30, 2006, which is hereby incorporated by reference for all purposes as if fully set forth herein.

BACKGROUND OF THE INVENTION

FIELD OF THE INVENTION

The present invention relates to a biosensor and, more particularly, to a biosensor which electrochemically measures an analyte contained in a bio sample.

DISCUSSION OF THE BACKGROUND

A biosensor consists of an electrode system and an enzyme reactant layer, in which the electrode system has a plurality of electrodes formed on an insulating substrate by screen printing, and the enzyme reactant layer is composed of hydrophilic polymer, oxidoreductase and electron acceptor which are formed on the electrode system. The biosensor electrochemically measures an analyte contained in a sample, which is disclosed in U.S. Patent Nos. 5,171,689 and 5,387,328.

In general, the biosensor includes a working electrode and a reference electrode. For instance, an electrochemical sensor measures an analyte with an oxidoreductase and an electron-transfer mediator according to the following reaction equation:

[Reaction equation]

Analyte + Enzyme (reduced) + Electron-transfer mediator (oxidized) → Product + Enzyme (oxidized) + Electron-transfer mediator (reduced)

The reaction equation shows that the reduced electron-transfer mediator which is produced by the reaction with the analyte contained in the sample is proportional to the concentration of the analyte contained in the sample. A predetermined voltage is applied to the working electrode with respect to the reference electrode to oxidize the reduced electron-transfer mediator. At this time, oxidation current is produced, and the amount of the analyte contained in the sample can be measured from the amount of the oxidation current.

When an oxidation reaction occurs in the working electrode made of carbon, a reduction reaction occurs in the reference electrode made of carbon. However, in the event the working electrode and the reference electrode are located closely to each other, the electron-transfer mediator (reduced) on the reference electrode may be diffused into the working electrode when a bio sample is injected to the enzyme reactant layer. That is, when the electrodes are located closely to each other, a measurement error occurs in the electrode due to an abnormal amount of oxidation current produced on the working electrode. Thus, the working electrode and reference electrode need to be separated from each other by a predetermined distance.

However, in the event the working electrode and the reference electrode are located far from each other, an exposed area of the substrate between the working electrode and the reference electrode becomes too large. In general, an enzyme solution is well coated on an electrode made of carbon, but is poorly coated on an insulating substrate since the insulating substrate has a greater surface tension to water than to carbon. Furthermore, since the insulating substrate is attached better to the electrode made of carbon than to the dried enzyme reactant layer, the dried enzyme reactant layer comes off from the substrate further than from the electrode. Accordingly, there is a problem in that the enzyme reactant layer formed on the electrodes may be washed away when the bio sample is injected.

SUMMARY OF THE INVENTION

The present invention provides a biosensor which enables a bio sample to be quickly absorbed and minimizes measurement errors by preventing an enzyme contained in the bio sample from being washed away from a working electrode or a reference electrode when the bio sample is injected to an enzyme reactant layer.

The present invention further provides a biosensor which prevents an enzyme reactant layer from coming off from a lower substrate or an insulating film.

Additional features of the invention will be set forth in the description which follows, and in part will be apparent from the description, or may be learned by practice of the invention.

The present invention discloses a biosensor measuring an analyte contained in a sample, including: a lower insulating substrate having formed thereon a working electrode and a reference electrode connected to lead terminals through leads, and an enzyme reactant layer formed on the electrodes to react with the analyte contained in the sample; a spacer which is interposed between the lower substrate and an upper substrate, is attached to the lower and upper substrates, and has a sample guide area to guide the sample to reach to the electrodes through the enzyme reactant layer; and an upper insulating substrate which faces the lower substrate through the spacer, where a dummy electrode is formed on the lower substrate, the dummy electrode being separated from the working electrode and the reference electrode, fixing the enzyme reactant layer, and being not connected to the leads.

Each of the electrodes may be made of carbon, graphite, platinum-carbon, silver, gold, palladium or platinum.

It is to be understood that both the foregoing general description and the following detailed description are exemplary and explanatory and are intended to provide further explanation of the invention as claimed.

BRIEF DESCRIPTION OF THE DRAWINGS

The accompanying drawings, which are included to provide a further understanding of the invention and are incorporated in and constitute a part of this specification, illustrate embodiments of the invention, and together with the description serve to explain the principles of the invention.

FIG. 1 is an exploded perspective view of a biosensor according to an exemplary embodiment of the present invention.
FIG. 2 is a partially plan view of a biosensor according to an exemplary embodiment of the present invention.
FIG. 3 is a plan view of a lower substrate according to an exemplary embodiment of the present invention.
FIG. 4 is a plan view of a lower substrate according to an exemplary embodiment of the present invention.
FIG. 5 is an exploded perspective view of a biosensor according to an exemplary embodiment of the present invention.
FIG. 6 is a cross-sectional view of a biosensor according to an exemplary embodiment of the present invention.
FIGS. 7 and 8 are enlarged views of part of a biosensor shown in FIG. 6 according to an exemplary embodiment of the present invention.
FIG. 9 is a view for illustrating the effect of a biosensor according to an exemplary embodiment of the invention.

DETAILED DESCRIPTION OF THE ILLUSTRATED EMBODIMENTS

The invention is described more fully hereinafter with reference to the accompanying drawings, in which exemplary embodiments of the invention are shown. This invention may, however, be embodied in many different forms and should not be construed as limited to the embodiments set forth herein. Rather, these embodiments are provided so that this disclosure is thorough, and will fully convey the scope of the invention to those skilled in the art. In the drawings, the size and relative sizes of layers and regions may be exaggerated for clarity. Like reference numerals in the drawings denote like elements.

It will be understood that when an element or layer is referred to as being "on" or "connected to" another element or layer, it can be directly on or directly connected to the other element or layer, or intervening elements or layers may be present. In contrast, when an element is referred to as being "directly on" or "directly connected to" another element or layer, there are no intervening elements or layers present.

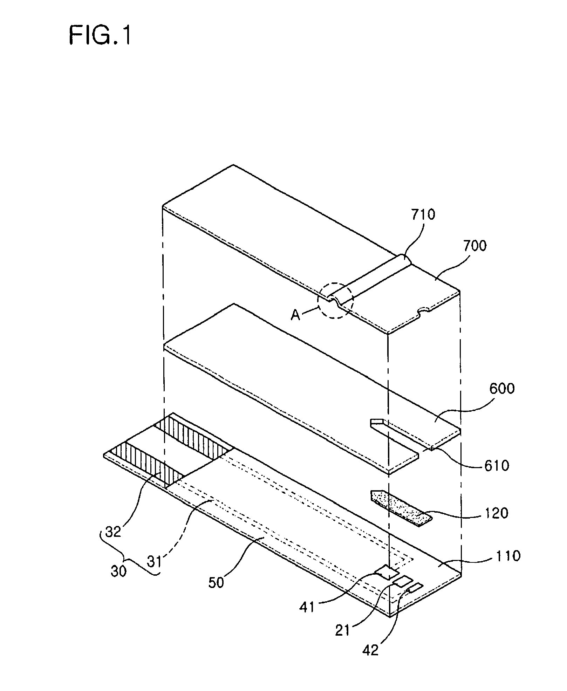

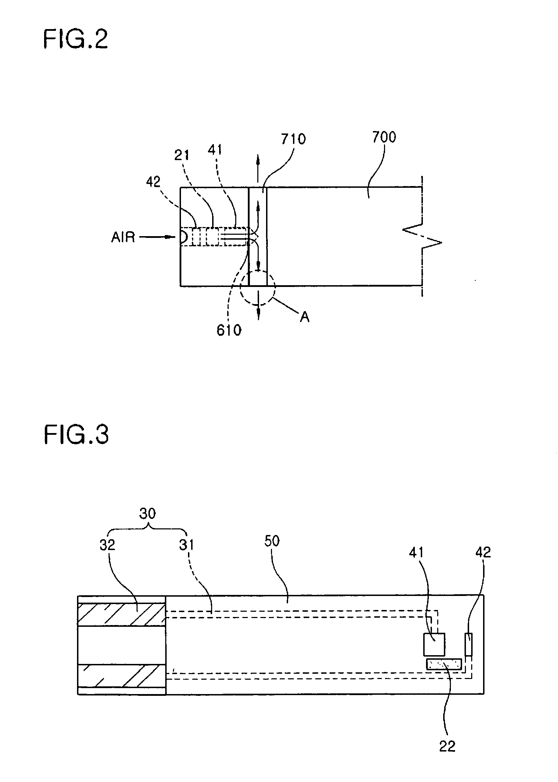

FIG. 1 is an exploded perspective view of a biosensor according to an exemplary embodiment of the present invention. FIG. 2 is a partially plan view of a biosensor according to an exemplary embodiment of the present invention.

The biosensor measures an analyte contained in a sample. The biosensor includes a lower insulating substrate 110, a spacer 600, and an upper insulating substrate 700.

The lower insulating substrate 110 has at least one electrode formed thereon, and an enzyme reactant layer formed on the electrode which reacts with an analyte contained in a sample. The spacer 600 is interposed between the lower and upper substrates 110 and 700 and is attached to the lower and upper substrates 110 and 700. The spacer 600 has a sample guide area 610 to guide the sample to reach to electrodes 21, 41 and 42 through an enzyme reactant layer 120. The upper insulating substrate 700 faces the lower substrate 600 and has an air discharge area 710 to discharge air which is absorbed together with the sample through the sample guide area 610. The air discharge area 710 is formed on a layer which is different from that of the sample guide area 610.

The air discharge area 710 is preferably formed in tunnel shape on a layer different from that of the sample guide area 610 to increase the absorption rate of the sample. The air discharge area 710 is preferably formed in semi-cylindrical shape in a direction perpendicular to the absorption direction of the sample.

The lower substrate 110 may be made of an insulating material with a thickness of 50 to 400um. The lower substrate 110 has a lead unit 30 formed thereon which includes leads 31 and lead terminals 32. The electrodes 21, 41 and 42 connected to the leads 31 are formed on the lower substrate 110 by screen printing. The electrodes 21, 41 and 42 are made of carbon, graphite, platinum-plated carbon, silver, gold, palladium or platinum. For example, an electrode may be printed on the lower substrate 110 using an ink composed of carbon or platinum-plated carbon, or an ink containing palladium.

The electrode 21 denotes a dummy electrode, 41 denotes a reference electrode, and 42 denotes a working electrode. The reference electrode 41 and the working electrode 42 detect the amount of current which is produced by the oxidation or reduction of an electron acceptor on an enzyme reactant layer 70. The dummy electrode 21 is separated from the working electrode 42 and the reference electrode 41. The dummy electrode 21 fixes the enzyme reactant layer 70 and is not connected to the lead. The dummy electrode 21 acts to prevent a dissolved enzyme contained in a bio sample injected to the enzyme reactant layer 70 from being washed away from the working electrode 42 or the reference electrode 41. In addition, the dummy electrode 21 acts to prevent the enzyme reactant layer 70 from becoming unfastened from the lower substrate 110 or insulating film 50 upon manufacturing the biosensor. The dummy electrode 21 may be formed between the working electrode 42 and the reference electrode 41.

The enzyme reactant layer 120 is made up of an enzyme, which reacts with the analyte contained in the sample, an electron-transfer mediator, which reacts with the enzyme, and a polymer holding substance, which fixes a buffer solution, an enzyme stabilizer, and the like on the electrode. The enzyme reactant layer 120 is covered and fixed on the electrodes 21, 41 and 42.

In this case, examples of the enzyme include oxidoreductase, such as glucose oxidase, lactate oxidase, cholesterol oxidase, and alcohol oxidase, transferase, such as glucose dehydrogenase, GOP (glutamate oxaloacetate transaminase, and GPT (glutamate pyruvate transaminase), and hydrolase.

Examples of the electron-transfer mediator include potassium ferricyanide, potassium ferrocyanide, hexaamineruthenium chloride, ferrocene and its derivative, and quinine and its derivative, which are substances reacting with the enzyme to be oxidized or reduced.

When a sample containing an analyte drops on the enzyme reactant layer 120, the enzyme reactant layer 120 is dissolved by the sample and the analyte contained in the sample reacts with an enzyme, thereby oxidizing the analyte and reducing the electron acceptor. After the enzyme reaction is finished, the amount of oxidation current which is obtained by electrochemically oxidizing the reduced electron acceptor is measured by a measurement apparatus (not shown) which contacts the leads 32 which are connected to the electrodes 41 and 42, thereby obtaining the concentration of the analyte contained in the sample.

The spacer 600 forms a capillary tube by the sample guide area 610 which is formed by attaching the upper and lower substrates 700 and 110 with each other. The spacer 600 has a thickness not thinner than the enzyme reactant layer 120 so that the sample can be easily injected to the enzyme reactant layer 120. That is, the sample is easily injected when the spacer 600 has a thickness greater than the enzyme reactant layer 120. The spacer 600 may be a double-sided adhesive tape with a thickness of 10 to 300um. More preferably, the spacer 600 has a double-sided adhesive tape with a thickness of 10 to 150um to minimize the amount of the injected sample. When the sample is automatically injected through the sample guide area 610 by capillary action, the air existing in the sample guide area 610 is discharged outside through the air discharge area 710 which is formed on the upper substrate 700.

The air discharge area 710 is preferably formed on a layer different from that of the sample guide area, more preferably in a tunnel shape, to increase the absorption rate of the sample. That is, the air discharge area 710 is formed on a layer on which the sample and the enzyme reactant layer 120 do not exist, such that only the air is discharged through the air discharge area 710. The air discharge area 710 is preferably formed in semi-cylindrical shape in a direction perpendicular to the absorption direction of the sample. The air discharge area 710 may be formed in other shapes.

When the sample starts to be injected to the enzyme reactant layer 120, the air existing around the enzyme reactant layer 120 is pushed toward the sample guide area 610, and is discharged outside through the air discharge area 710 which is formed on the upper substrate 700 in a direction perpendicular to the injection direction of the sample. That is, since only the air is discharged outside through the air discharge area 710 which is formed in a tunnel shape on a layer different from that of the sample guide area 610, the absorption rate of the sample increases.

That is, since the air discharge area 710 is formed on a layer different from that of that of the enzyme reactant layer 120, the amount of the sample is irrelevant to the dimension of the air discharge area 710. Accordingly, the amount of the sample can be reduced as much as the dimension of the enzyme reactant layer 120. In addition, since the air discharge area 710 can be increased irrespective of the dimension of the enzyme reactant layer 120, it is possible to efficiently discharge the air. Furthermore, since the air is discharged without passing through the enzyme reactant layer 120, the discharge rate of the air is irrelevant to the absorption rate of the sample. As a result, the discharge rate of the air increases, causing the absorption rate of the sample to increase. Moreover, when the biosensor is removed from the measurement apparatus, a user's hands are rarely stained with the sample.

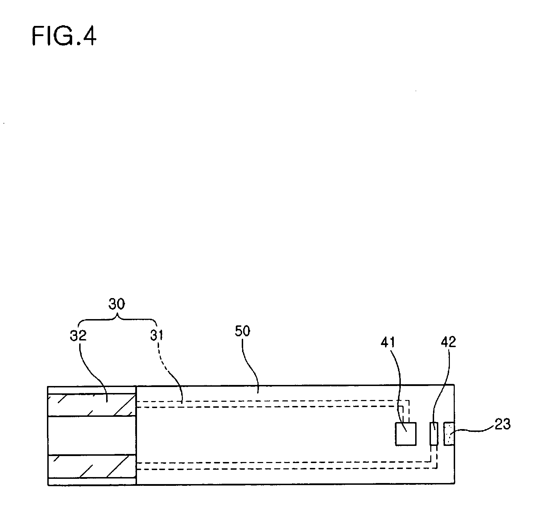

FIGS. 3 and 4 are plan views of a lower substrate having a dummy electrode formed thereon according to exemplary embodiments of the present invention. While a single dummy electrode is used in the present embodiment, two or more electrodes may be used. In addition, while a single working electrode and a single reference electrode are used in the embodiment, a dummy electrode may be formed on the lower substrate in a biosensor having two working electrodes.

As shown in FIG. 3, a dummy electrode 22 may be formed to be adjacent to the working electrode 42 and the reference electrode 41. As shown in FIG. 4, a dummy electrode 23 may be formed on an end portion of the lower substrate on which the enzyme reactant layer 70 is formed. Since the dummy electrode 23 which is firmly attached to the enzyme reactant layer 70 is formed on a surface of the biosensor which is cut, it is possible to reduce the possibility that the enzyme reactant layer can be broken when the biosensor is cut.

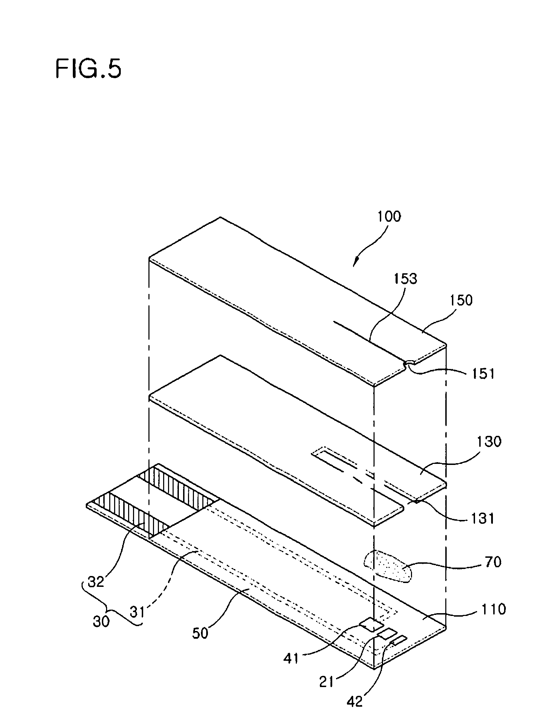

FIG. 5 is an exploded perspective view of a biosensor according to an exemplary embodiment of the invention. The biosensor 100 includes a lower substrate 110, a spacer 130, and an upper substrate 150.

The lower substrate 110 may be made of an insulating material with a thickness of 50 to 400um. The lower substrate 110 has a lead unit 30 formed thereon which includes leads 31 and lead terminals 32. The electrodes 21, 41 and 42 connected to the leads 31 are formed on the lower substrate 110 by screen printing. The electrodes 21, 41 and 42 are made of carbon, graphite, platinum-plated carbon, silver, gold, palladium or platinum. For example, an electrode may be printed on the lower substrate 110 using an ink composed of carbon or platinum-plated carbon, or an ink containing palladium.

The electrode 21 denotes a dummy electrode, 41 denotes a reference electrode, and 42 denotes a working electrode. The reference electrode 41 and the working electrode 42 detect the amount of current which is produced by the oxidation or reduction of an electron acceptor on an enzyme reactant layer 70. The dummy electrode 21 is separated from the working electrode 42 and the reference electrode 41. The dummy electrode 21 fixes the enzyme reactant layer 70 and is not connected to the lead. The dummy electrode 21 act to prevent a dissolved enzyme contained in a bio sample injected to the enzyme reactant layer 70 from being washed away from the working electrode 42 or the reference electrode 41. In addition, the dummy electrode 21 acts to prevent the enzyme reactant layer 70 from becoming unfastened from the lower substrate 110 or insulating film 50 upon manufacturing the biosensor. The dummy electrode 21 may be formed between the working electrode 42 and the reference electrode 41.

The enzyme reactant layer 120 is applied on the electrodes 21, 41 and 42 which are formed on the lower substrate 110. The enzyme reactant layer 120 is made up of an enzyme, which reacts with the analyte contained in the sample, an electron-transfer mediator, which reacts with the enzyme, and a polymer holding substance, which fixes a buffer solution, an enzyme stabilizer, and the like on the electrode.

The spacer 130 is interposed between the lower and upper substrates 110 and 150 and is attached to the lower and upper substrates 110 and 150. The spacer 130 has a sample guide area 310 to guide the sample to reach to electrodes 21, 41 and 42 through an enzyme reactant layer 70.

The upper substrate 150 may be formed of a thin plate made of PET, PVC or polycarbonate. The upper substrate 150 has an air outlet 153 to discharge air within the sample guide area 131 which is formed by the spacer 130, so that a sample (e.g., blood) can be injected to the enzyme reactant layer 70 by capillary action. The air outlet 153 is formed to be extended from a curved portion 151 onto the electrodes 21, 41 and 42. The curved portion 151 is curved towards the electrodes 21, 41 and 42 from an end of the upper substrate 150.

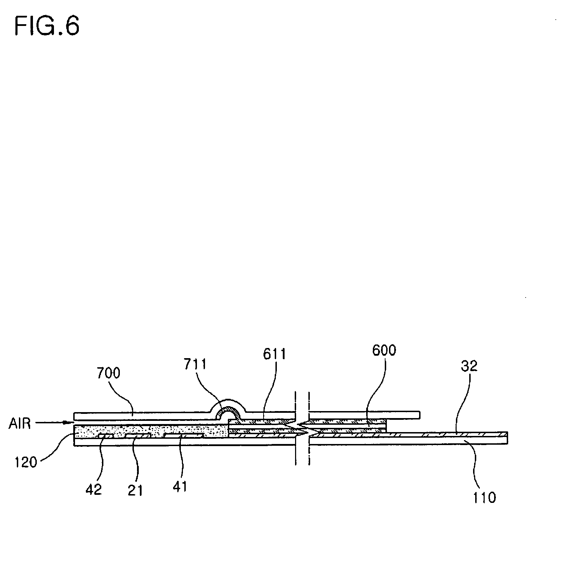

FIG. 6 is a cross-sectional view of a biosensor according to an exemplary embodiment of the invention.

The electrodes 21, 41 and 42 are formed on the lower substrate 110 on which the enzyme reactant layer 120 is fixed. The lower substrate 110 is combined with the upper substrate 700 by the spacer 600 having the sample guide area 610. The upper substrate 700 has the air discharge area 710 formed thereon in tunnel shape in a direction perpendicular to the injection direction of the sample to discharge air as the sample is injected through the sample guide area 610.

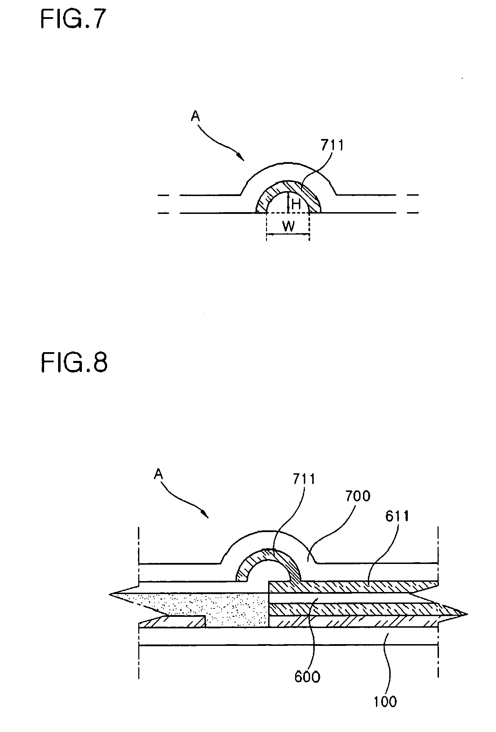

The present invention provides a biosensor which quickly discharges only air through the air discharge area 710 and prevents the sample from running out. FIGS. 7 and 8 are enlarged views of part of the biosensor shown in FIG. 6.

As a first solution to prevent the sample from running out, the air discharge area 710 is appropriately limited in width and height. In the event the air discharge area is so small in cross-section, the sample may run out by capillary action. Accordingly, the larger cross-section the air discharge area 710 has, the higher the discharge rate of the air and the absorption rate of the sample. However, it is not possible to unlimitedly increase the air discharge area 710. Since the air discharge area is formed in tunnel shape, it is difficult to form the air discharge area having too large cross-section, and the air discharge area may be easily deformed after it is formed.

Our experiments showed that the air discharge area 710 preferably has a cross-section with a width of about 0.3 to 3mm and a height of about 0.1 to 3mm. At this time, the amount of the sample injected through the sample guide area is preferably less than about 1$\mu$l.

As a second solution to prevent the sample from running out, the inner wall of the air discharge area 710 is applied with a hydrophobic material since the sample and the enzyme reactant layer 120 have a hydrophilic property.

As shown in FIGS. 7 and 8, the inner wall of the air discharge area is preferably coated with a hydrophobic material to prevent the hydrophilic sample from flowing out toward the air discharge area 710. The spacer 600 also is preferably made of a hydrophobic material. In addition, surfaces of the spacer 600 which contact the upper and lower substrates are preferably applied with hydrophobic adhesives.

That is, the enzyme reactant layer 120 is made of a hydrophobic material so that the hydrophilic sample cannot flow out of the enzyme reactant layer 120.

As a third solution to prevent the sample from running out, the sample guide area 610 has an end portion which is tapered to a point.

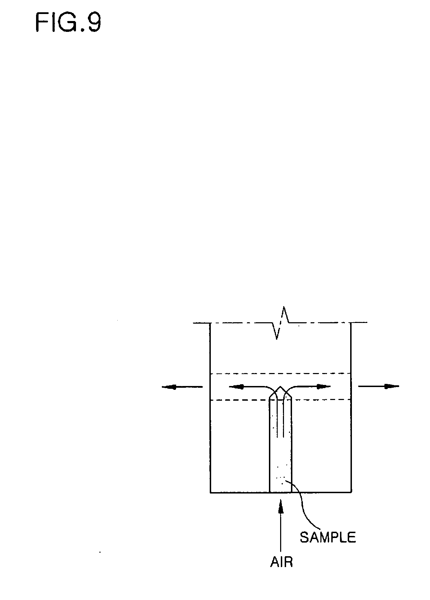

FIG. 9 is a view for illustrating the effect of a biosensor according to an exemplary embodiment of the invention.

Since the injected sample remains within the sample guide area 610 and the enzyme reactant layer 120, the sample does not flow out. Accordingly, it is possible to minimize the amount of the sample used in the measurement. At the same time, only the air is discharged through the air discharge area 710 which is formed on a layer which is different from that of the sample guide area 610. Accordingly, it is possible to provide a biosensor which can increase the absorption rate of the sample and prevent the sample from flowing out.

As apparent from the above description, a bio sample can quickly run into the biosensor since the sample guide area is open through the air outlet of the upper substrate.

In addition, since the dummy electrode, together with the working electrode and the reference electrode, which fixes the enzyme reactant layer, is formed on the lower substrate, it is possible to prevent the dissolved enzyme contained in the bio sample injected to the enzyme reactant layer from being washed away from the working electrode or the reference electrode. In addition, when the biosensor is manufactured, it is possible to prevent the enzyme reactant layer from becoming unfastened from the lower substrate or the insulating film. In addition, since the dummy electrode is made of the same material as the working electrode or the reference electrode, an additional process is not necessary to form the dummy electrode on the lower substrate.

In addition, since the air discharge area is formed on the upper substrate on a layer different from that of the sample injection area in a direction perpendicular to the injection direction of the sample, it is possible to efficiently discharge air upon absorbing the sample, and thus to increase the absorption rate of the sample. In addition, the present invention provides various solutions to prevent the sample from running out through the air discharge area.

It will be apparent to those skilled in the art that various modifications and variation can be made in the present invention without departing from the spirit or scope of the invention. Thus, it is intended that the present invention cover the modifications and variations of this invention provided they come within the scope of the appended claims and their equivalents.

**Claims**

1. A biosensor measuring an analyte contained in a sample, comprising:

   a lower insulating substrate having formed thereon a working electrode and a reference electrode connected to lead terminals through leads, and an enzyme reactant layer formed on the electrodes to react with the analyte contained in the sample;
   a spacer which is interposed between the lower substrate and an upper substrate, is attached to the lower and upper substrates, and has a sample guide area to guide the sample to reach to the electrodes through the enzyme reactant layer; and
   an upper insulating substrate which faces the lower substrate through the spacer,

   wherein a dummy electrode is formed on the lower substrate, the dummy electrode being separated from the working electrode and the reference electrode, fixing the enzyme reactant layer, and being not connected to the leads.

2. The biosensor of claim 1, wherein each of the electrodes is made of carbon, graphite, platinum-carbon, silver, gold, palladium or platinum.

3. The biosensor of claim 1, wherein the dummy electrode is formed between the working electrode and the reference electrode.

4. The biosensor of claim 1, wherein the dummy electrode is formed to be adjacent to the working electrode or the reference electrode.

5. The biosensor of claim 1, wherein the dummy electrode is formed on an end portion of the lower substrate on which the enzyme reactant layer is formed.

6. The biosensor of claim 1, wherein an insulating film is formed on the lower substrate to insulate the working electrode, reference electrode and dummy electrode from one another.

7. The biosensor of claim 1, wherein an air discharge area is formed on the upper substrate to discharge air which is absorbed together with the sample through the sample guide area formed on the spacer.

FIG.1

## FIG.2

## FIG.3

FIG.4

FIG.5

FIG.6

FIG.7

A

711

H

W

FIG.8

A

711

700

611

600

100

# FIG.9

SAMPLE

AIR

**REFERENCES CITED IN THE DESCRIPTION**

**Patent documents cited in the description**

- KR 2006120043
- US 5171689 A
- US 5387328 A